# FASCICULE DE BREVET EUROPEEN

(11) **EP 1 613 291 B1**
(45) Date de publication et mention de la délivrance du brevet: **26.01.2011**
(21) Numéro de dépôt: 04742453.6
(22) Date de dépôt: 07.04.2004
(51) Int. Cl.: A61K 8/73, A61Q 19/08

(54) **COMPOSITION NON-THÉRAPEUTIQUE DE DEUX POLYSACCHARIDES A BASE DE FUCOSE ET DE RHAMNOSE**
NICHTTHERAPEUTISCHE ZUSAMMENSETZUNGEN MIT ZWEI POLYSACCHARIDEN AUF BASIS VON FUCOSE UND RHAMNOSE
NON-THERAPEUTIC COMPOSITION OF TWO POLYSACCHARIDES BASED ON FUCOSE AND RHAMNOSE

(30) Priorité: 08.04.2003 FR 0304336
(43) Date de publication de la demande: 11.01.2006
(73) Titulaire: Industria E Comércio de Cosméticos Natura Ltda., CEP-0007750-000 Cajamar, SP (BR)
(72) Inventeur: GESZTESI, Jean-Luc, Sao Paulo (BR); VILLA, Nova, Silva, Luciana, Sao Paulo (BR); ROBERT, Ladislas, F-94440 Santeny (FR); ROBERT, Alexandre, Michel, F-94440 Santeny (FR)
(74) Mandataire: Texier, Christian
(86) Numéro de dépôt international: PCT/FR2004/000864
(87) Numéro de publication internationale: WO 2004/091568

(56) Documents cités:
- WO-A-2004/078789
- DE-A- 19 503 423
- DE-A- 19 704 693
- FR-A- 2 652 742
- FR-A- 2 813 789
- FR-A- 2 821 554

## Description

La présente invention concerne une nouvelle composition cosmétique de deux polysaccharides à base de fucose et de rhamnose et son utilisation notamment dans des produits à application topique, pour lesquels une activité sur le tissu cutané épithélial et conjonctif est recherchée, en particulier des produits cosmétiques à effet anti-âge.

Au cours du vieillissement la peau s'amincit d'environ 7% en moyenne tous les dix ans (Skin thickness changes in normal aging skin, Branchet et al., Gerontology, 1990, 36: 28-35). Cet amincissement cutané concerne l'épiderme et le derme. Cette perte de tissu représente une perte approximativement équivalente de cellules, kératinocytes de l'épiderme et des fibroblastes du derme. Les kératinocytes présents dans l'épiderme, ainsi que les fibroblastes présents dans le derme sont connus pour élaborer des enzymes protéolytiques qui contribuent à un important amincissement cutané (Dr L. ROBERT: Le vieillissement, CNRS, Belin, 1994; Dr L. ROBERT: Le vieillissement, faits et théories, Dominos, Flammarion, 1995).

Cependant, différentes macromolécules de la matrice extracellulaire (ECM) se comportent différemment. La perte des fibres de collagène a été montrée comme étant moins rapide que la perte de tissu de peau totale. A l'inverse, la biosynthèse de fibronectine a été montrée comme augmentant dans la peau avec l'âge et par des fibroblastes en culture en fonction du nombre de passages. Les fibres élastiques du derme papillaire, formant un réseau vertical, sont progressivement dégradées. Une majorité des réseaux de fibres élastiques horizontales prédominent avec l'âge dans le derme. La densité de surface des fibres élastiques, quantifiée par analyse d'image, a été montrée comme augmentant avec l'âge. Cette nette augmentation de la densité de surface de l'élastine avec l'âge est cependant accompagnée par un déclin progressif de l'élasticité de la peau. Ces données montrent la complexité des modifications dépendantes de l'âge, du tissu de la peau et de sa composition macromoléculaire.

L'un des paramètres mentionnés ci-dessus concerne l'épaisseur de la peau qui diminue avec l'âge. Les deux couches de derme et de l'épiderme sont affectées par cette évolution.

Ces dernières années, de nombreuses recherches ont eu pour objet d'obtenir des composés actifs contre certains effets du vieillissement cutané. Un premier objectif est d'obtenir un épaississement de la peau. Un autre objectif est de ralentir ce processus d'amincissement cutané et de restaurer l'épaisseur normale du derme.

Les saccharides et les polysaccharides sont des substances bien connues en cosmétique, notamment pour leurs propriétés hydratantes.

Les RROP-s (rhamnose rich oligo- and polysaccharides) sont utilisés en solution aqueuse à 2,5% (p/p) de polysaccharides de haut poids moléculaire composés à 50% de rhamnose. Les RROP-s agissent sur l'adhésion aux kératinocytes au niveau des lectines à rhamnose et sur l'inhibition de l'adhésion des polynucléaires aux kératinocytes. Les RROPs permettent donc de moduler la propagation des messages cellulaires et d'atténuer par conséquence les réactions irritatives consécutives (effet anti-inflammatoire).

Les FROP-s (fucose rich oligo- and polysaccharides) sont des oligo- et polysaccharides composés de polymères d'un trisaccharide contenant du galactose, de l'acide acétyl-galacturonique et du fucose, qui agit sur les fibroblastes du derme en stimulant leur prolifération, en les protégeant contre la cytotoxicité induite par les radicaux libres émis par l'ascorbate en présence de Fe et d'EDTA (Demandes de brevet FR 2813885 et FR 2813789).

Le document DE-19704693 décrit une composition cosmétique pour les soins de la peau (augmente la 'vitalité de la peau') contenant un mélange un polysaccharide à unité fucose, comme par exemple le Fucogel 1000, et rhamnose oû son polysaccharide naturel: Gummi arabicum. Dans le Gummi arabicum polysaccharide il y a seulement 13% de rhamnose (1/8 parts) (voir tabelle I et p.2,1.24-27).

Le document FR-2652742 une composition anallergique cosmétique pour traiter ou améliorer les propriétés élastiques de la peau et accélérer la régénération de l'épiderme. Les compositions contiennent des monosaccharides comme le fucose, galactose et rhamnose. Lesdits monosaccharides peuvent être intégrés dans une structure polysaccharide, sans précisés les pourcentages des chaque monosaccharides (voir p.21.10-19 et revendications).

On a maintenant constaté de manière surprenante et inattendue qu'un nouveau mélange de composé rhamnosique et de composé fucosique présente des activités significatives sur différents composants de la peau, se traduisant par un véritable résultat anti-vieillissement nettement visible. Cet effet anti-vieillissement de la composition tient aux propriétés complémentaires des RROP-s agissant sur les kératinocytes en surface de la peau et des FROP-s agissant sur les fibroblastes du derme, notamment par l'inhibition de la dégradation par les radicaux libres de l'acide hyaluronique qui assure dans le derme une fonction fondamentale de remplissage et de cohésion de la peau. L'effet anti-vieillissement de la composition se traduit également par l'augmentation de l'épaisseur du derme et de l'épiderme.

### Description

Ainsi la présente invention a pour objet l'utilisation non-thérapeutique d'une composition destinée à une action anti-vieillissement de la peau caractérisée en ce qu'elle comprend au moins un composé rhamnosique RROP-s, un compose fucosique FROP-s et un excipient cosmétiquement acceptable, le composé rhamnosique RROP-s étant un oligo ou un polysaccharide composé à 50% de rhamnose et le composé fucosique FROP-s étant un oligo ou polysaccharide composé de polymères d'un trisaccharide contenant du galactose, de l'acide acétyl galacturonique et du fucose.

La composition cosmétique selon la présente invention peut présenter des pourcentages variables de composé fucosique FROP-s et de composé rhamnosique RROP-s, avec en général une majorité de composé fucosique FROP-s, pouvant aller jusqu'à une composition à parties égales de composé fucosique FROP-s et de composé rhamnosique RROP-s. De préférence, le mélange de polysaccharides selon l'invention comprend 1/10 de composé rhamnosique et 9/10 de composé fucosique.

Plus particulièrement, le mélange de polysaccharides selon l'invention comprent 1/5 de composé rhamnosique et 4/5 de composé fucosique. Plus particulièrement le mélange de polysaccharides selon l'invention comprend 1/3 de composé rhamnosique et 2/3 de composé fucosique.

Plus particulièrement encore, le mélange de polysaccharides selon l'invention comprend 1/2 de composé rhamnosique et 1/2 de composé fucosique.

Le mélange de polysaccharides est tout particulièrement approprié comme actif dans une composition cosmétique notamment anti-âge (application topique). En particulier, les effets biologiques constatés de ce mélange de polysaccharides s'avèrent comparables voire supérieurs à ceux des polysaccharides pris séparément, leurs actions s'étant révélées complémentaires et synergiques.

L'excipient cosmétiquement acceptable peut être tout excipient parmi ceux connus de l'homme du métier en vue d'obtenir une composition selon l'invention sous forme d'une crème, d'une lotion, d'un gel, d'une pommade, etc., éventuellement sous la forme d'une émulsion avec en outre des composants connus de l'homme du métier, pour améliorer, modifier ou stabiliser la composition d'un point de vue cosmétique.

A titre d'exemple, la composition selon l'invention peut comprendre des excipients bien connus de l'homme du métier pour la formulation de composition destinée à une application topique. De tels excipients peuvent être choisis parmi le groupe constitué par les agents structurants de la peau (tels que le squalène et les sphingolipides), les agents humectants (tels que la glycérine et l'hydroxy prosilan C), les émollients (tels que le butylène glycol et le lactate de cétyl), les silicones (telles que la cyclométhicone), les agents de protection solaire (tels que les parsol 1789 et Eusolex 6300), les émulsifiants (notamment le Carbopol 1342 associé à la triéthanolamine et la lécithine de soja), les épaississants (notamment la gomme de xanthane), les agents séquestrants (notamment l'EDTA), les antioxydants (tels que BHT), les fragrances, les conservateurs, l'eau et les mélanges de ceux-ci.

Le mélange d'oligosaccharides est utilisé selon une proportion comprise entre environ 0,1 et 10% en poids, par rapport au poids total de la composition cosmétique.

Bien entendu, les conditions opératoires pour préparer la composition cosmétique selon l'invention font partie des connaissances générales de l'homme du métier.

### Légende des figures

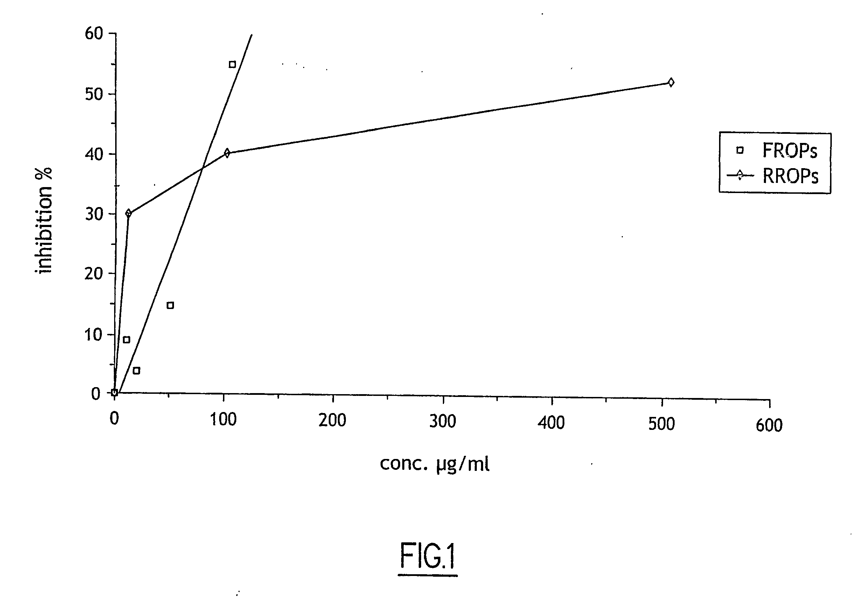

La Figure 1 est une courbe reportant les résultats d'efficacité de capture des radicaux libres par les FROP-s et les RROP-s présentés à l'exemple 1, en terme de pourcentage d'inhibition de la dégradation par les radicaux libres de l'acide hyaluronique sur des explants de peau par concentration en FROP-s et en RROP-s.

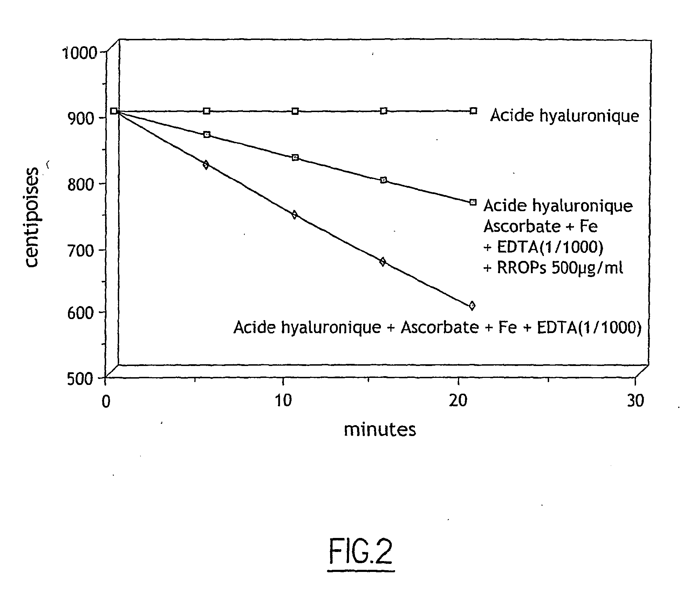

La Figure 2 est une courbe présentant les résultats de dégradation de l'acide hyaluronique en présence de radicaux libres et/ou de RROP-s présentés à l'exemple 2, en utilisant une mesure viscosimétrique basée sur la libération de radicaux libres (°OH) par l'ascorbate en présence de Fe et EDTA.

### Exemple1

Dans cet exemple on a comparé la capacité de capture des radicaux libres des RROP-s et des FROP-s en utilisant une mesure viscosimétrique basée sur la libération de radicaux libres (°OH) par l'ascorbate en présence de Fe et EDTA.

### Figure 1

La Figure 1 présente l'inhibition dose-dépendante par les FROP-s et les RROP-s de la dégradation par les radicaux libres de l'acide hyaluronique.

On observe que les RROP-s présentent une importante inhibition à faible concentration (moins de 10 µg/ml) mais que cet effet arrive à un plateau à des plus fortes concentrations, après 100 µg/ml.

On observe que les FROP-s présentent une faible inhibition pour les faibles concentrations (moins de 20% d'inhibition jusqu'à 50 µg/ml). Cependant, l'inhibition augmente de façon linéaire à de plus fortes concentrations et dépasse l'inhibition observée pour les RROP-s: à 100 µg/ml l'inhibition est de 38% pour les RROP-s et de 55% pour les FROP-s. En conclusion, ces résultats suggèrent qu'aux concentrations relatives à la composition du mélange de FROP-s et de RROP-s de la présente invention, il y a un effet synergique de ces deux polysaccharides sur la capture des radicaux libres.

### Exemple 2

Dans cet exemple on a mesuré la variation de la viscosité de l'acide hyaluronique en présence de radicaux libres et/ou de RROP-s en utilisant une mesure viscosimétrique basée sur la libération de radicaux libres (°OH) par l'ascorbate en présence de Fe et EDTA.

### Figure 2

La Figure 2 présente la dégradation de l'acide hyaluronique par les radicaux libres libérés par l'ascorbate en présence de Fe et EDTA en fonction du temps d'exposition: 1) en l'absence d'agent libérant des radicaux libres, 2) en présence de tels agents à 1/1000 et 3) en présence de tels agents à 1/1000 et de RROP-s à 500 µg/ml.

On observe que la variation de viscosité entre l'acide hyaluronique seul et en présence de radicaux libres est de 14,5 cpoises/min, alors qu'elle n'est que de 7,0 cpoises/min en présence de RROP-s. Le pourcentage d'inhibition par les RROP-s de la dégradation de l'acide hyaluronique par l'agent libérant des radicaux libres est donc de 52%.

On en conclut que les RROP-s ont une action de protection de l'acide hyaluronique vis-à-vis des radicaux libres.

### Exemple 3

Dans cette exemple nous avons examiné les effets d'une application locale de RROP-s (Rhamnosoft^{®}) et de FROP-s (Elastinol^{®}) et le mélange des deux sur l'épaisseur de la peau du rat sans poils.

### MATERIEL ET METHODES

### Animaux et traitements

10 rats femelles sans poils d'un poids initial moyen de 170g ont été utilisés pour cette étude. Ces 10 animaux sont divisés en 3 groupes:
le premier groupe inclut 3 rats (n°-s 1, 2 et 3)
le second groupe inclut également 3 rats (n°-s 4, 5 et 6), et finalement
le troisième groupe comprend 4 rats (n°-s 7, 8, 9 et 10).

Les rats sont gardés dans des cages individuelles et ont un accès libre à l'eau et à de la nourriture industrielle pour rats. Le côté gauche de ces animaux est utilisé pour le contrôle et traité uniquement par la préparation de base utilisée comme véhicule: BioDerma "Biobase Crème H/E". Le côté droit des animaux est traité avec la même base contenant également les principes actifs suivants: Rhamnosoft 0,25% pour le premier groupe, Elastinol 0,75% pour le deuxième groupe et mélange des deux polysaccharides dans les mêmes proportions, donnant une concentration finale de 1 % pour le troisième groupe. 1 g de ces trois préparations est administré localement 5 jours par semaine pendant 4 semaines. La pénétration de ces préparations est effectuée par étalement du produit et friction pendant approximativement une minute de chaque côté.

### Collection des échantillons de peau

Après la période de traitement les animaux sont sacrifiés par injection d'une dose létale d'anesthésique au pentobarbital. Afin d'éviter la contraction de la peau après prélèvement, on colle sur la peau des rats des anneaux de plastique de 10 mm de diamètre en utilisant de la colle cyanoacrylate (cyanolithe). La peau est ensuite découpée autour de l'anneau (0,785 cm²) et prélevée. On colle un second anneau sur la surface intérieure de la peau, exactement à l'opposé de l'autre anneau. Ces échantillons de peau circulaires sont ensuite fixés dans une solution de Bouin pendant 24 heures suivi d'un lavage, d'une déshydratation, d'une imprégnation de solvants de la paraffine, puis on pratique l'inclusion dans de la paraffine contenant un polymère synthétique (paraplast). De chaque bloc de paraplast on confectionne 12 coupes d'une épaisseur de 5µ avec un microtome Reichert. Deux de ces coupes sont colorées avec l'hématoxyline et l'éosine (HE), deux colorants histologiques. De telles coupes colorées à l'HE sont utilisées pour la mesure de l'épaisseur du derme.

### Evaluation de l'épaisseur du derme

Cette évaluation se fait sur les coupes colorées à l'hématoxytine-éosine, observées sur un photo-microscope Zeiss muni d'une caméra vidéo noir et blanc, relié à un microordinateur Nixdorf Power Tower contenant le logiciel d'analyse d'image Visiolab 1000 de Biocom (France). On utilise la fonction de mesure de longueur semi-automatique, les résultats sont exprimés en pixels (points image). Le rapport d'agrandissement est de 20x. La calibration est faite en mesurant des longueurs connues sur une cellule de Malassez, ce qui permet la conversion des valeurs en pixels en microns. Pour obtenir l'épaisseur du derme, des droites perpendiculaires sont tracées de la lame basale dermo-épidermique jusqu'à la limite supérieure de l'hypoderme. 10 champs microscopiques sélectionnés de façon aléatoire ont été analysés pour chaque échantillon de peau, avec 25 mesures par champ, ce qui donne un total de 250 mesures pour chaque échantillon. Les mesures individuelles, ainsi que les moyennes ± la déviation standard de chaque champ sont enregistrés et servent à l'évaluation finale des résultats.

Comme tous les 10 rats ont un côté contrôle, les valeurs témoins sont les moyennes de 2500 mesures individuelles. Comme seulement 3 rats composent les deux premiers groupes, les moyennes pour ceux-ci sont obtenues sur 750 mesures. Finalement, pour le groupe traité avec le mélange des deux polysaccharides, la moyenne est basée sur 1000 mesures individuelles. Un nombre aussi élevé de mesures confère un haut degré de sécurité à l'évaluation statistique des résultats, effectuée avec le logiciel StatView et le test t de Student.

### Résultat de l'épaisseur du derme

Pour les mesures de l'épaisseur du derme un objectif de 2,5x est utilisé, donnant un grossissement final de 20x. Le Tableau 2 montre les résultats obtenus, exprimés en pixels (points image).

**Tableau 1**

| Traitement des rats | Valeur moyenne en pixels | Erreur standard à la moyenne | test t de Student comparativement aux contrôles |
|---|---|---|---|
| Contrôles | 172,21 | 4,67 | |
| Rhamnosoft | 182,96 | 5,52 | N.S. |
| Elastinol | 179,96 | 5,63 | N.S. |
| Mélange Elastinol/Rhamnosoft | **199,19** | 2,88 | p<0,0001 |

On voit que les trois moyennes correspondant aux dermes des échantillons de peaux traitées sont plus ou moins supérieures à la valeur témoin. L'épaisseur moyenne des dermes témoins est de 172,21±4,67 pixels.

Pour les dermes traités au Rhamnosoft, le chiffre correspondant est de 182,96±5,52 pixels. Ceci correspond à une augmentation de l'épaisseur du derme de 6,2% par rapport à la valeur témoin, mais cette différence n'est pas statistiquement significative.

Pour les dermes traités à l'Elastinol, l'épaisseur moyenne obtenue est de 179,96±5,63 pixels, supérieur de 4,5% seulement à la valeur témoin. Cette différence n'est pas non plus statistiquement significative.

L'épaisseur moyenne du derme après 4 semaines de traitement avec un mélange de Rhamnosoft et de l'Elastinol est de 199,19±2,88 pixels. Cette augmentation de l'épaisseur du derme traité par ce mélange, comparée avec la valeur témoin, est statistiquement hautement significative: p<0,0001.

### DISCUSSION

Les résultats exposés précédemment indiquent que dans des concentrations faibles ni le Rhamnosoft (0,25%) ni l'Elastinol (0,75%) seuls, n'augmentent significativement l'épaisseur du derme de la peau des rats traités. A l'inverse, quand le mélange des deux de ces polysaccharides est utilisé pour le traitement, chacun étant présent à la concentration individuellement utilisée, une augmentation statistiquement significative de l'épaisseur du derme est observée, correspondant à peu près à 16% de l'épaisseur du derme témoin. Cette augmentation est supérieure à la somme des effets individuels des deux polysaccharides testés (6,2+4,5=10,7) de pratiquement 50%, et ceci suggère que les deux substances testées potentialisent leurs actions, ou plus simplement qu'elles ont un effet synergique sur l'épaisseur du derme.

### Exemple 4

Nous avons montré dans l'exemple précédent que l'association des FROP-s et des RROP-s permet une augmentation statistiquement significativement élevée de l'épaisseur du derme. Dans le présent exemple nous avons comparé par morphométrie semi-automatique l'effet sur la densité cellulaire de l'épiderme des FROP-s (Elastinol^{®}) et des RROP-s (Rhamnosoft^{®}) ou du mélange des deux.

### MATERIEL ET METHODES

### Expérimentation animale

### Animaux et traitements

10 rats femelles sans poils d'un poids initial de 170 g ont été utilisés. Ils ont été gardés dans des cages individuelles, avec accès libre à l'eau et à de la nourriture industrielle pour rats. Le côté gauche des animaux a été utilisé comme contrôle et traité uniquement avec la préparation de base utilisée comme véhicule. Le côté droit a été traité différemment dans les trois groupes de rats.
Le premier groupe, rats n° 1, 2 et 3: Rhamnosoft 0,25%
Le deuxième groupe, rats n° 4, 5 et 6: Elastinol 0,75%
Le troisième groupe, rats n° 7, 8, 9 et 10: Rhamnosoft 0,25%+Elastinol 0,75%

1 g de ces préparations a été administré localement, 5 jours par semaine pendant 4 semaines. La pénétration de ces préparations a été assurée par étalement et friction pendant approximativement une minute de chaque côté.

### Collection des échantillons de peau

Après la période d'administration les animaux sont sacrifiés par injection d'une dose létale d'anesthésique au pentobarbital. Afin d'éviter la contraction de la peau après prélèvement, on colle sur la peau des rats des anneaux de plastique de 10 mm de diamètre en utilisant de la colle cyanoacrylate (cyanolithe). La peau est ensuite découpée autour de l'anneau (0,785 cm²) et prélevée. On colle un second anneau sur la surface intérieure de la peau, exactement à l'opposé de l'autre anneau. Ces échantillons de peau circulaires sont ensuite fixés dans une solution de Bouin pendant 24 heures suivi d'un lavage, d'une déshydratation, d'une imprégnation de solvants de la paraffine, puis on pratique l'inclusion dans da la paraffine contenant un polymère synthétique (paraplast). De chaque bloc de paraplast 12 coupes de 5µ ont été préparées avec un microtome Reichert, et montées sur lames. Deux d'entre elles ont été colorées avec de l'hématoxyline-éosine (HE), deux colorants histologiques. De telles coupes à l'HE ont été utilisées pour la mesure de la densité cellulaire de l'épiderme.

### Evaluation de la densité cellulaire

Cette évaluation se fait sur les coupes colorées à l'hématoxyline-éosine, observées sur un photo-microscope Zeiss muni d'une caméra vidéo noir et blanc, relié à un microordinateur Nixdorf Power Tower contenant le logiciel d'analyse d'image Visiolab 1000 de Biocom (France). On utilise la fonction d'extraction manuelle des contours, les résultats sont exprimés en pixels (points image). Le rapport d'agrandissement est de 320x. En premier lieu nous avons mesuré la surface explorée, exprimée en pixels. Ensuite nous avons extrait manuellement tous les périmètres de toutes les cellules présentes dans le champ microscopique. Le logiciel a calculé à partir de ces mesures pour chaque champ le nombre de cellules, la surface de chaque cellule et le nombre de pixels contenant une cellule, ainsi que les moyennes de ces valeurs pour chaque échantillon étudié. 10 champs microscopiques sélectionnés de façon aléatoire ont été analysés pour chaque échantillon de peau. L'évaluation statistique des résultats a été effectuée avec le logiciel StatView et le test t de Student.

### Résultats

Les résultats obtenus, exprimés en pixels, sont donnés dans les Tableaux 3, 4 et 5, un par traitement administré. 4 paramètres sont indiqués dans ces tableaux. Le premier est la surface explorée (en pixels ± l'erreur standard à la moyenne). Le deuxième donne le nombre de cellules observées sur la surface explorée. Le troisième paramètre est la surface, toujours exprimée en pixels, qui contient une cellule, donnant ainsi la possibilité d'exprimer numériquement la densité des cellules de l'échantillon étudié. Finalement, la variation de la surface moyenne des cellules peut indiquer un effet trophique éventuel sur les cellules.

**Tableau 2.**

| Résultats obtenus sur des rats traités par le Rhamnosoft (N°-s 1, 2 et 3). | | | | |
|---|---|---|---|---|
| | surfaces explorées | n cellules | pixels par cellule | surf. moyenne des cellules |
| Contrôles | 50909±1820 | 31,7±1,27 | 1648,07± | 415,6±11,65 |
| Rhamnosoft | 64279±4609 | 42,36±2,97 | 1529±65,23 | 494,93±20,38 |
| test t | p<0,002 | p<0,000 | p<0,1+61 N.S. | p<0,001 |

On voit sur le tableau que dans les coupes des peaux traitées avec le Rhamnosoft le nombre des cellules est significativement supérieur au nombre correspondant des témoins. Il en va de même pour la surface moyenne des cellules. Par contre le nombre de pixels contenant une cellule ne diffère pas significativement entre traités et témoins. Ceci signifie, que la cellularité de l'épiderme traité au Rhamnosoft est du même ordre, que celle de l'épiderme de contrôle. Il apparaît par contre un effet trophique significatif sur les cellules dans la peau traitée.

**Tableau 3.**

| Résultats exprimés en pixels obtenus pour les rats traités à l'Elastinol (N°-s 4, 5 et6) | | | | |
|---|---|---|---|---|
| | surface explorée | n cellules | pixels par cellule | surf.moyenne des cellules |
| Contrôles | 50909±1820 | 31,7±1,27 | 1648,07± | 415,6±11,65 |
| Elastinol | 56987±6931 | 36,00±2,78 | 1556,02±79,61 | 468,53±31,8 |
| test t | p<0,227N.S. | p<0,145 N.S. | p<,360 N.S. | p<0,000 |

Les résultats de mesure représentés dans le Tableau 4 ne montrent pas de différences statistiquement significatives entre les témoins et les échantillons traités à l'Elastinol, ni pour la surface explorée, ni pour le nombre de cellules comptées sur la surface explorée, ni pour le nombre de pixels contenant une cellule. Seule la différence de la surface moyenne des cellules est significative: échantillons traités: 468,53 contre témoin: 415,60; p<0,000.

**Tableau 4.**

| Résultats exprimés en pixels obtenus pour les rats traités à l'Elastinol+Rhamnosoft (N°-s 7, 8, 9, 10) | | | | |
|---|---|---|---|---|
| | Surface explorée | n cellules | pixels par cellule | Surf.moyenne des cellules |
| Contrôles | 50909±1820 | 31,7±1,27 | 1648,07±45,6 | 415,6±11,65 |
| Elast+Rhamno | 53853±2612 | 34,44±1,51 | 1623,80±111,1 | 497,88±23,53 |
| test t | p<0,387 N.S. | p<0,236 N.S. | p<0,810 N.S. | p<0,001 |

Comme on peut l'observer sur ce Tableau, les différences entre le témoin et les rats traités à l'Elastinol+Rhamnosoft ne sont pas significatives pour les trois premiers paramètres. Seule la surface moyenne d'une cellule est significativement supérieure pour les échantillons traités par rapport aux témoins.

### DISCUSSION, CONCLUSION

Considérant que seuls les résultats statistiquement différents des témoins peuvent être retenus, on observe que seul l'étude de la surface moyenne des cellules correspond à ce critère, avec l'application de Rhamnosoft seul et l'application de l'association Rhamnosoft+Elastinol. C'est encore le mélange des deux polysaccharides (RROP-s et FROP-s) qui permet d'obtenir l'augmentation la plus importante de la surface moyenne des cellules, donc l'effet anti-vieillissement de l'épiderme le plus important.

## Revendications

1. Utilisation d'une composition cosmétique destinée à une action anti-vieillissement de la peau **caractérisée en ce qu'**elle comprend au moins un composé rhamnosique RROP-s, un composé fucosique FROP-s et un excipient cosmétiquement acceptable, le composé rhamnosique RROP-s étant un oligo ou un polysaccharide composé à 50% de rhamnose et le composé fucosique FROP-s étant un oligo ou polysaccharide composé de polymères d'un trisaccharide contenant du galactose, de l'acide acétyl galacturonique et du fucose.

2. Utilisation selon la revendication 1, **caractérisée en ce que** la composition comprend, par rapport au poids total du mélange des polysaccharides 1/10 de composé rhamnosique RROP-s et 9/10 de composé fucosique FROP-s.

3. Utilisation selon la revendication 1, **caractérisée en ce que** la composition comprend, par rapport au poids total du mélange des polysaccharides 1/5 de composé rhamnosique RROP-s et 4/5 de composé fucosique FROP-s.

4. Utilisation selon la revendication 1, **caractérisée en ce que** la composition comprend, par rapport au poids total du mélange des polysaccharides 1/3 de composé rhamnosique RROP-s et 2/3 de composé fucosique FROP-s.

5. Utilisation selon la revendication 1, **caractérisée en ce que** la composition comprend, par rapport au poids total du mélange des polysaccharides 1/2 de composé rhamnosique RROP-s et 1/2 de composé fucosique FROP-s.

6. Utilisation selon la revendication 1 **caractérisée en ce que** la composition est adaptée à une application topique, notamment **en ce qu'**elle se présente sous la forme d'une crème.

7. Utilisation selon l'une quelconque des revendications 1 à 6 **caractérisée en ce que** le mélange d'oligosaccharides est utilisé selon une proportion comprise entre environ 0.1 et 10% en poids, par rapport au poids total de la composition.

## Claims

1. Use of a cosmetic composition intended for antiaging action on the skin, **characterized in that** it comprises at least one rhamnose compound RROPs, one fucose compound FROPs and a cosmetically acceptable excipient, the rhamnose compound RROP-s being an oligosaccharide or a polysaccharide composed of 50% of rhamnose, and the fucose compound FROP-s being an oligosaccharide or a polysaccharide composed of polymers of a trisaccharide containing galactose, acetylgalacturonic acid and fucose.

2. Use according to Claim 1, **characterized in that** the composition comprises, relative to the total weight of the polysaccharide mixture, 1/10 of rhamnose compound RROPs and 9/10 of fucose compound FROPs.

3. Use according to Claim 1, **characterized in that** the composition comprises, relative to the total weight of the polysaccharide mixture, 1/5 of rhamnose compound RROPs and 4/5 of fucose compound FROPs.

4. Use according to Claim 1, **characterized in that** the composition comprises, relative to the total weight of the polysaccharide mixture, 1/3 of rhamnose compound RROPs and 2/3 of fucose compound FROPs.

5. Use according to Claim 1, **characterized in that** the composition comprises, relative to the total weight of the polysaccharide mixture, 1/2 of rhamnose compound RROPs and 1/2 of fucose compound FROPs.

6. Use according to Claim 1, **characterized in that** the composition is suitable for topical application, especially **in that** it is in the form of a cream.

7. Use according to any one of Claims 1 to 6, **characterized in that** the oligosaccharide mixture is used in a proportion of between about 0.1% and 10% by weight relative to the total weight of the composition.

## Patentansprüche

1. Verwendung einer kosmetischen Zusammensetzung, die für eine Anti-Age-Wirkung der Haut bestimmt ist, **dadurch gekennzeichnet, dass** sie mindestens eine Rhamnoseverbindung RROP-s, eine Fucoseverbindung FROP-s und einen kosmetisch akzeptablen Exzipienten umfasst, wobei die Rhamnoseverbindung RPOP-s ein Oligo- oder ein Polysaccharid ist, das zu 50 % aus Rhamnose besteht und die Fucoseverbindung FROP-s ein Oligo- oder Polysaccharid ist, das aus Polymeren eines Trisaccharids besteht, das Galaktose, Acetylgalakturonsäure und Fucose enthält.

2. Verwendung nach Anspruch 1, **dadurch gekennzeichnet, dass** die Zusammensetzung im Verhältnis zum Gesamtgewicht des Polysaccharidgemischs 1/10 Rhamnoseverbindung RROP-s und 9/10 Fucoseverbindung FROP-s umfasst.

3. Verwendung nach Anspruch 1, **dadurch gekennzeichnet, dass** die Zusammensetzung im Verhältnis zum Gesamtgewicht des Polysaccharidgemischs 1/5 Rhamnoseverbindung RROP-s und 4/5 Fucoseverbindung FROP-s umfasst.

4. Verwendung nach Anspruch 1, **dadurch gekennzeichnet, dass** die Zusammensetzung im Verhältnis zum Gesamtgewicht des Polysaccharidgemischs 1/3 Rhamnoseverbindung RROP-s und 2/3 Fucoseverbindung FROP-s umfasst.

5. Verwendung nach Anspruch 1, **dadurch gekennzeichnet, dass** die Zusammensetzung im Verhältnis zum Gesamtgewicht des Polysaccharidgemischs 1/2 Rhamnoseverbindung RROP-s und 1/2 Fucoseverbindung FROP-s umfasst.

6. Verwendung nach Anspruch 1, **dadurch gekennzeichnet, dass** die Zusammensetzung für eine topische Anwendung geeignet ist, vor allem **dadurch**, dass sie sich in Form einer Creme darstellt.

7. Verwendung nach einem der Ansprüche 1 bis 6, **dadurch gekennzeichnet, dass** das Oligosaccharidgemisch gemäß einem Verhältnis zwischen zirka 0,1 und 10 Gew.-% inklusive in Bezug zum Gesamtgewicht der Zusammensetzung verwendet wird.
